# EUROPEAN PATENT SPECIFICATION

(11) **EP 0 426 273 B1**
(45) Date of publication and mention of the grant of the patent: **12.10.1994**
(21) Application number: 90305590.3
(22) Date of filing: 23.05.1990
(51) Int. Cl.: F04B 43/08, A61M 5/142

(54) **Pumping device**
Pumpenvorrichtung
Dispositif de pompage

(30) Priority: 02.11.1989 GB 8924766
(43) Date of publication of application: 08.05.1991
(73) Proprietor: BAXTER INTERNATIONAL INC., Deerfield, IL 60015-4633 (US)
(72) Inventor: Faulkner, Eric A, Dr., Maidenhead, Berks. SL6 1TS (GB); Danby, Hal, Sudbury, Suffolk C010 0PZ (GB)
(74) Representative: MacGregor, Gordon

(56) References cited:
- DE-A- 2 939 212
- US-A- 3 918 854
- US-A- 4 781 548

## Description

This invention relates to pumping devices particularly for use in medical applications such as the intravenous supply of fluids to a patient.

DE-A-2939212 discloses a pump comprising a plurality of separate pressure elements, each element comprising a first, second and third member and a counter pressure surface. The first member is positioned opposite the counter pressure surface, and the second member is positioned opposite the third member. The members and counter pressure surface define a channel in which tubing is received. The first member is driven by a cam and is movable towards and away from the counter pressure surface. In order to effect pumping of fluid through the tubing, the first member is moved towards the surface, thereby deforming the tubing in a first radial direction. The first member then moves away from the counter pressure surface as the second and third members move towards each other, thereby squeezing the tubing in a radial direction that is perpendicular to the first direction. In this way, the normal cross-sectional shape of the tubing is restored.

Reformation of the tubing in order to restore the normal cross-sectional shape of the tubing is desirable, because tubing tends to display memory loss when repeatedly deformed in the same direction. Hence, pumping accuracy is reduced as pumping proceeds, because the original volume of the tubing will not be attained between times when tubing is deformed if the tubing is not restored to its original cross-sectional shape between these deformations.

The pre-characterising parts of Claims 1 and 2 are based on DE-A-2939212, and the distinguishing features of the present invention are set out in the characterising parts of Claims 1 and 2.

The pumping apparatus of the present invention has a simple operation, since it only requires reciprocation of two members in order to deform and then reform the tubing. No cam is required.

According to the present invention there is provided pumping apparatus comprising a length of tubing for providing passage of liquid through the apparatus, first and second members defining a chamber receiving the tubing, the members being relatively reciprocable to cause alternate deformation and reformation of the tubing in a pumping action wherein the volume of the tubing is reduced when it is deformed, the apparatus including means for urging the tubing to its reformed state in which the normal cross-sectional shape of the tubing is restored,
characterised in that, in a first relative position of the members, the chamber is substantially cylindrical, whereby the members urge the tubing to its reformed state, the members being relatively movable in a direction radial of the chamber and tubing to a second relative position, in which the chamber is elongate to cause deformation of the tubing, and controllable valve means are provided upstream and downstream of the members to control the flow of liquid pumped through the tubing.

According to the present invention there is also provided a device for use in such a pumping apparatus, the device comprising first and second members for deforming and reforming the length of tubing.

Reference below to deforming means is to be construed as a reference to the first and second members.

Preferably the valve means on the input and the output sides are such that when fully operated, flow is stopped or permitted, as the case may be.

Preferably the arrangement is such that deformation of said tubing by said deforming means is non-occlusive, that is to say that at the extreme of deformation the opposite internal surfaces of the tubing which approach each other do not make contact.

In some examples of devices in accordance with the invention the deforming means is such that deformation in said other direction ceases as the original cross-sectional shape of the tubing is regained. In other examples the squeezing means is such that squeezing in said other direction continues beyond restoration of the original cross-sectional shape of said tubing to cause further local deformation of the cross-sectional shape of said tubing.

Preferably said deforming means comprises two members each having a valley along which said tubing may pass, one of said two members being inverted relative to the other with their valleys together forming a passage for said tubing through said deforming means and said two members being arranged to move to and fro relative to one another in a direction transverse to the direction of said passage whereby to deform said tubing as aforesaid.

Preferably said deforming means comprises two members each having a series of transverse blades or ridges shaped to provide a valley through which said tubing may pass, one of said members being inverted relative to the other with its ridges interdigitated with the ridges of the other, said two members being arranged to move relative to one another in a direction transverse to the direction of passage of said tubing through said two valleys whereby to deform said tubing.

Preferably each ridge has a recess which is generally semi-circular to one side and of progressively decreasing depth to the other side until the full height of the ridge is reached.

Preferably all of the ridges of one member are substantially identical, with the generally semi-circular portions of their apertures to the same side.

Preferably again viewed in the direction of passage of the tubing through the valley formed by the ridges, all of the ridges of one member appear superimposed.

With an arrangement as just described the generally semi-circular portions of the apertures in the ridges of one member are to one side and the generally semi-circular portions of the apertures in the ridges of the relatively inverted member are to the other side, as viewed in the direction of passage of said tubing through the valley.

Where, as will normally be the case, the length of tubing, when undeformed, is of substantially constant circular section through the pumping device, the curvature of the generally semi-circular portions of the apertures in the ridges of both members are normally such that in one position of relative movement of the two members, the generally semi-circular portions of the apertures in the ridges of the two members together form a passage of substantially circular cross-section of diameter closely similar to that of said tube.

Preferably said formed passage is of diameter slightly less than that of said tubing whereby gently to nip said tubing.

Preferably the ridges of each member bear on the surfaces between the ridges of the other member.

Preferably said two members are biassed one towards the other by resilient means, e.g. a spring.

In a preferred embodiment one of said two members is arranged to be stationary during operation whilst the other moves in the manner of a shuttle.

Where the pumping apparatus comprises a housing with a closure, such as a door or lid, preferably the member which is arranged to be stationary during operation is carried by said closure whereby opening said closure releases said tubing from said deforming means. With such an arrangement, preferably the member which is arranged to be stationary during operation has limited freedom to move, independent of said closure, towards and away from said other member, biassing means, such as a spring, being provided between it and said closure tending to urge it towards said other member.

Preferably said member which is arranged to move in the manner of a shuttle is arranged to be driven via an eccentric by an electric motor which is controlled to move in operation in a series of discrete steps producing incremental steps of said member in a direction producing deformation of said tubing.

Preferably said last-mentioned member is arranged to be returned by said motor in the opposite direction in one relatively rapid movement.

Preferably each valve means comprises a guide member having a channel therethrough for said tubing and, within said channel, a rotary member having an off-centre projection extending generally parallel to the axis of rotation of said rotary member and having one face against which said tubing lies, the arrangement being such that said face defines, in part, said channel and partial rotation of said rotary member causes said tubing to be occluded by the resultant action of said face upon said tubing.

Preferably said face of said projection is partially recessed with a profiled surface adapted to bear on said tubing when said rotary member is partially rotated to a "start" position whilst the resulting overhang provides a closure (which may be partial) over the channel in said guide member capturing said tubing therein. Preferably stop means are provided for each rotary member whereby movement is limited in one direction of rotation to a position in which the face of its projection is so aligned with the tubing as to permit said tubing to be removed from said channel and in the other direction of rotation to a position beyond that at which said tubing is occluded. Preferably the control of said rotary members is such that in normal operation said last-mentioned position beyond that at which said tubing is occluded is not reached.

Preferably the stops in each case are provided by an arcuate slot or recess in the rotary member and co-axial with its axis of rotation, in co-operation with a fixed pin or other abutment.

Preferably the two valve means are controlled to be operated without, or substantially without, overlap between the periods at which each is permitting liquid flow. In other words, movement of the rotary member of one valve means in a direction to reduce flow is arranged to be completed, or substantially completed, before movement of the rotary member of the other valve means in a direction to increase flow, and vice versa.

With an arrangement as just described, normally operator controllable means are provided for causing the rotary members of both valve means to rotate to a position in which the faces of said projections are so aligned with the tubing and the deforming means is so relaxed as to permit the tubing to be removed from said channel.

Where said pumping device comprises a housing with a closure, such as a door or lid, preferably opening said closure is arranged to cause both valve means to be set to conditions restricting the flow of liquid and preferably thereafter to cause the moving member of said deforming means to be returned to a position of minimum deformation of said tubing.

Preferably again the arrangement is such that closing said closure causes the moving member of said deforming means to be driven to its extreme position of movement in a direction deforming said tube with the valve means on the outlet side in a condition of restricted flow and the valve means on the inlet side in a condition of increased flow and thereafter indexed back, with both valve means remaining in the conditions just mentioned, to a predetermined start position whereafter the valve on the inlet side is set to a condition of restricted flow and the valve on the outlet side is set to a condition of increased flow and the cycle of operation of said deforming means and valve means is commenced.

Preferably said aforementioned operator controllable means comprises a control (e.g. a button) accessible to an operator only when said closure is open and operable after the sequence of operations consequent upon opening said closure, as described above, is complete. Before operating said aforementioned operator controllable means an operator may have closed a clamp (e.g. a roller clamp) fitted to the tubing in order to avoid passage of fluid through said tubing when the length of tubing is removed from said channel.

Preferably each rotary member and the moving member of said deforming means are arranged to be driven by dedicated electric motors.

Preferably the control means controlling the operation of the motors driving said rotary members and the motor driving the member of said deforming means which is arranged to move in the manner of a shuttle comprises a microprocessor. Preferably at least the motor arranged to drive the movable member of the deforming means has associated therewith an encoder which produces an output signal indicative of the position of or extent to which the member driven by that motor has moved, means being provided for passing the signals thus produced to said microprocessor for use as reference signals in the timing of the generation of motor control signals by said microprocessor.

The microprocessor and its associated control electronics may be housed within said housing or remotely therefrom with cable or other suitable interconnection.

By suitably selecting the timing of the movements of the rotary members and the moving member of the deforming means and the increments by which the last-mentioned is driven, it is possible to achieve a satisfactorily smooth and consistent flow of liquid through the pumping device as required for the intravenous supply of fluids to a patient for example. For such purposes it is important that the tubing be readily disposable and, as will be appreciated, the construction of a pumping apparatus in accordance with the present invention may be such that the tubing may be changed rapidly when required whilst avoiding uncontrolled flow of fluid to the patient.

Normally the tubing used is standard p.v.c. tubing, in a typical medical application of diameter approximately 4.1mm and wall thickness of 0.5mm.

The invention is illustrated in and further described with reference to the accompanying drawings in which:-
Figure 1 illustrates in highly schematic fashion the cycle of operation of one simple form of pumping apparatus in accordance with the present invention.
Figure 2 illustrates, semi-schematically, a preferred form of pumping apparatus in accordance with the present invention intended for medical applications such as the intravenous supplies of fluid to a patient.
Figure 3 illustrates in greater detail the inlet and outlet valve means 3 and 4 of Figure 2.
Figures 4 and 5 illustrate the nature and operation of the deforming means 2 of Figure 2.
Figure 6 illustrates the method of driving the moving or shuttle member 27 of Figure 4.
Figure 7 is a semi-schematic perspective view of a complete pumping device as described with reference to Figures 2 to 6.
Figure 8 shows in section the door 41 of Figure 7 together with the stationary member 26 and moving member 27 of the deforming means illustrated in Figure 4.
Figure 9 is a side view partly in cross-section of another embodiment of the invention, and
Figure 10 is an end view partly in cross-section along the line III-III of Figure 9.

In all of the Figures, parts are not necessarily represented to scale.

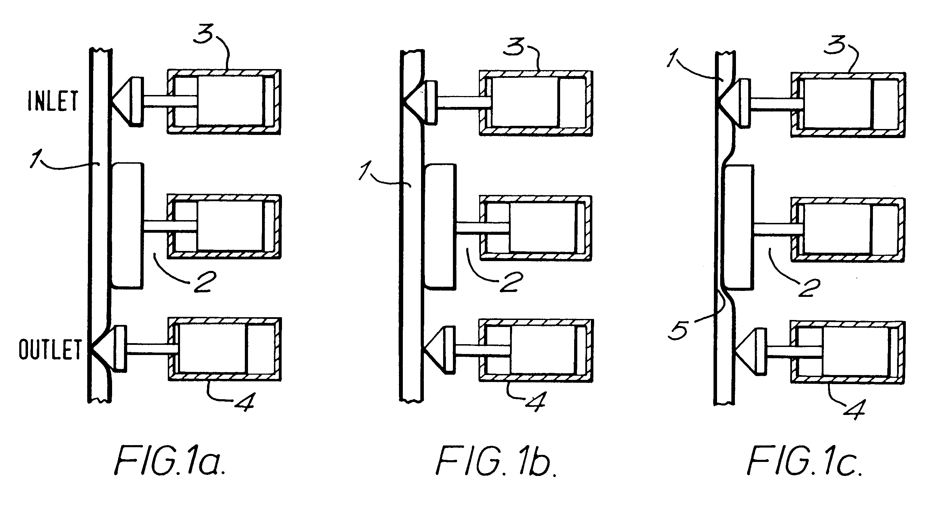

Referring to Figure 1, this illustrates in highly schematic manner at (a), (b) and (c), the three principle stages in a cycle of operation of one simple form of pumping apparatus in accordance with the present invention.

In (a), (b) and (c) a length of flexible p.v.c. tubing is represented at 1. The p.v.c. tubing is standard tubing of substantially constant undeformed cross-sectional dimensions throughout its length. Means for locally deforming the tubing 1 by squeezing is shown at 2, whilst on both the inlet (top as viewed) and outlet (bottom as viewed) sides of the squeezing means 2 are controllable valve means, 3 and 4 respectively, for restricting (and in this case shutting off by occlusion) the flow of liquid in the tube 1.

In Figure 1(a) the outlet valve means 4 is activated to close off flow to the outlet. The deforming means 2 is relaxed, and the inlet valve means 3 is relaxed thus permitting flow from the inlet.

In Figure 1(b) outlet valve means 4 has relaxed and inlet valve means 3 has been activated to close off the inlet. Deforming means 2 is about to be activated.

In Figure 1(c) deforming means 2 is shown fully activated, with outlet valve means 4 remaining relaxed and inlet valve means 3 remaining activated. Fluid now passes to the outlet. It may be noted that even when deforming means 2 is fully activated as shown in (c) the tubing 1 is not occluded, there remaining a small gap 5 between approaching opposite sides of the tubing deformed by squeezing.

The cycle then repeats save that, whilst not represented in the simple representation of Figure 1, following the deforming action illustrated in (c) the tubing would be deformed in a different direction tending to restore the original cross-sectional shape of the tubing.

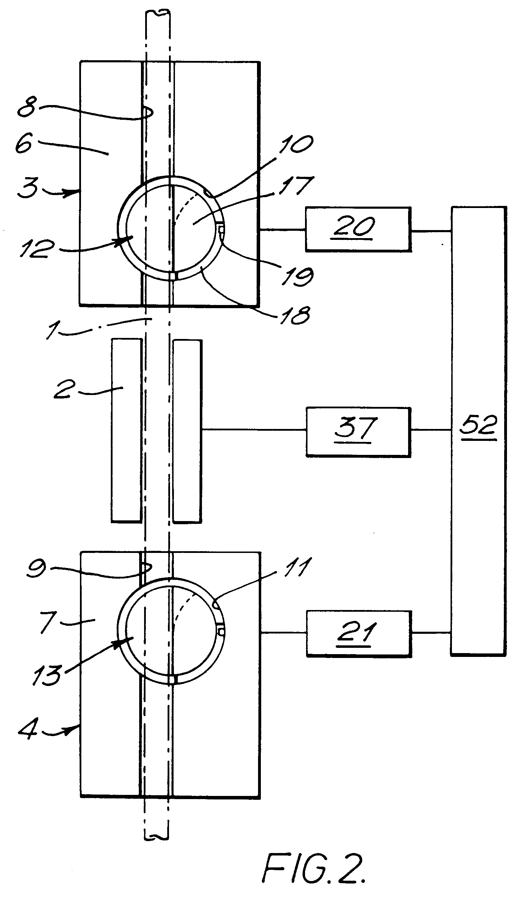

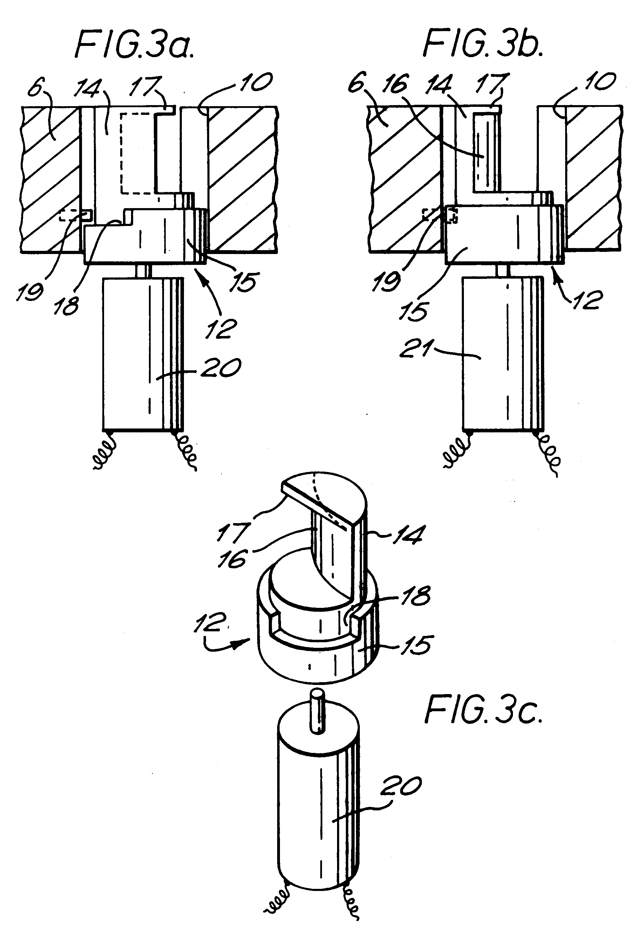

Referring to Figure 2, further details of the inlet valve means 3 and outlet valve means 4 and the squeezing means 2 are shown in Figures 3 and Figures 4 and 5 respectively.

In Figure 2 the tubing 1 is shown in dotted outline. Each of the valve means 3, 4 consists of a guide member 6, 7 having a channel 8, 9 in which the tubing 1 may rest. Within each channel 8, 9 is a circular enlargement 10, 11 housing a rotary member 12, 13. Further understanding of the nature of the arrangement may be gained by reference to Figures 3(a), (b) and (c) of which Figures 3(a) and (b) illustrate a transverse section across the guide member 6 through the centre of the rotary member 12, viewed in the direction of liquid flow from the inlet (top as viewed) to the outlet (bottom as viewed) and Figure 3(c) is a perspective view of rotary member 12 removed from the circular enlargement within channel 8. Whilst only the arrangement of rotary member 12 is shown in and described with reference to Figures 3(a), (b) and (c), the arrangement of rotary member 13 may be taken to be essentially similar. Rotary member 12 has a projection 14 extending into the channel 8 from a base portion 15. Projection 14 is off-centre to accommodate the tubing 1. The projection 14 is formed with a progressively recessed profiled surface 16 which acts upon the surface of the tubing 1 as rotary member 12 is rotated in an anti-clockwise direction (as viewed in Figure 2). The recession formed by the profiling of the surface 16, leaves an overhang 17. When rotary member 12 is rotated as described, overhang 17 provides a partial closure over channel 8 which renders the tubing 1 captive. As shown in Figure 2, and in (b) of Figure 3 the rotation of rotary member 12 is such that the surface 16 is, broadly speaking, aligned with the channel 8 such that the tubing is not captive. In this state, the tubing may readily be removed (ignoring the effects of the deforming means 2 for the moment and assuming that rotary member 13 is similarly rotated). If rotary member 12 is rotated in an anti-clockwise direction (as viewed in Figure 2) the profiled surface 16 bears upon the tubing 1 and this occludes the tubing 2 at that point and acts to shut off the inlet (corresponding rotation of rotary member 13 shuts off the outlet of course).

In fact, the rotary members 12, 13 are only rotated to positions shown in Figure 2 and Figure 3(b) when the pumping device is inoperative and an operator has operated a control to set them thus, so as to enable the tubing 1 to be discarded and replaced by fresh tubing. Normally the "start" position for each rotary member 12, 13 in its cycle of operation is one as represented in Figure 3(a) for member 12. The rotary member in question is rotated (again anti-clockwise as viewed in Figure 2) until the overhang 17 (in the case of rotary member 12) covers the channel 14 sufficient to prevent accidental removal of the tubing 1, and the tube is nipped almost to occlusion. For medical applications, as referred to, the amount of flow required is small (typically 100cc's per hour) and the actual rotation required of the rotary member from a position at which the tubing 1 is occluded to a position permitting sufficient flow is correspondingly not great.

Each rotary member 12, 13 is connected to be rotated by d.c. motors 20, 21 to and fro over a predetermined arc from the "start" position of rotation as aforesaid to a position in which the tubing is occluded by the profiled surface, 16 in the case of rotary member 12. In order to provide stops limiting rotational movement of the rotary members 12, 13 in each direction of rotation, arcuate recesses (18 in the case of rotary member 12 as shown in Figure 3(c)) are provided in the base portions (15 in the case of member 12) of each rotary member 12,13. These arcuate recesses co-operate with fixed pins such as that schematically represented at 19 in Figures 3(a) and (b).

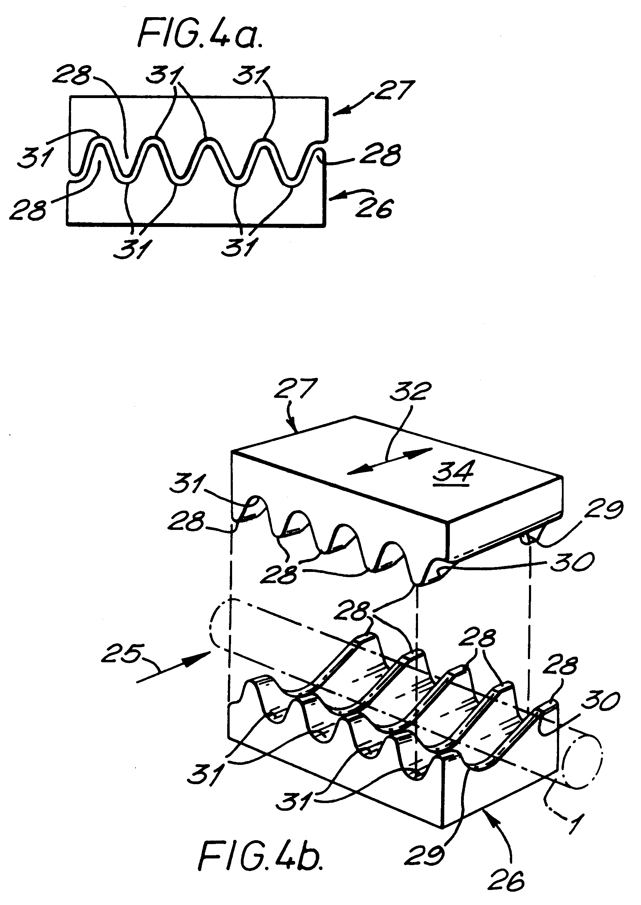

Referring to Figure 4 this illustrates at (b), by way of a perspective sketch, the two principal components of the deforming means 2 of Figure 2. The view shown in (a) is in the direction of the arrow 25 in (b) and shows the two principal component members 26 and 27 of the deforming means united.

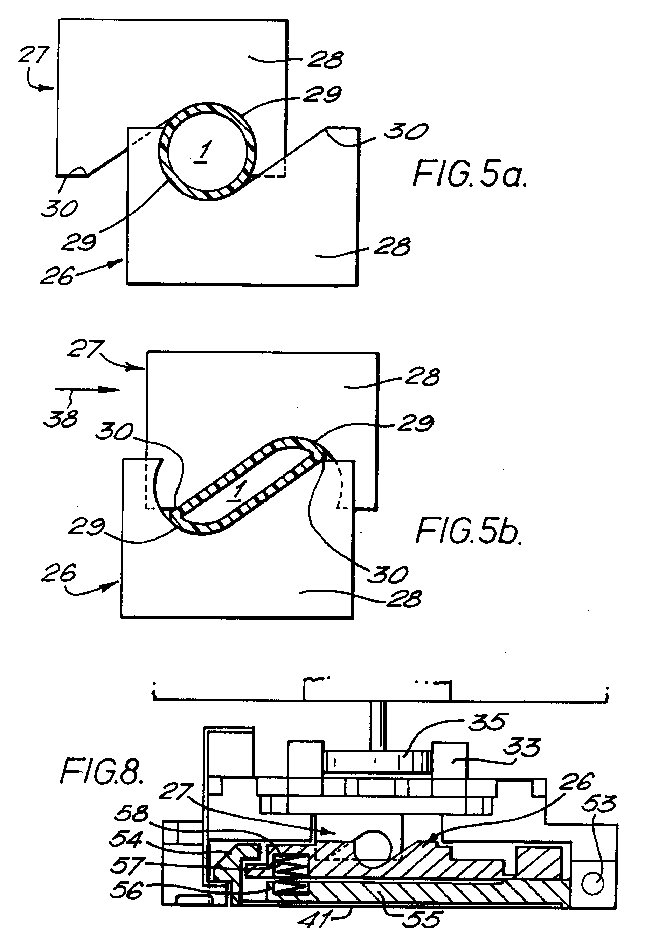

Member 26 has a series of transverse ridges 28 which are shaped to provide a valley through which the aforementioned tubing 1, again shown in dotted outlined may pass. As best illustrated in Figure 5, which demonstrates the action of the deforming means 2, each ridge 28 has a recess which is semi-circular to one side 29 and of progressively decreasing depth towards the other side 30 of the recess until the full height of the ridge is reached. Viewed in the direction of the passage of the tubing 1 through the valley formed by the recesses in the ridges 28 all of the ridges of the member 26 appear superimposed one upon the other, with all of the semi-circular portions of the recesses to the same side.

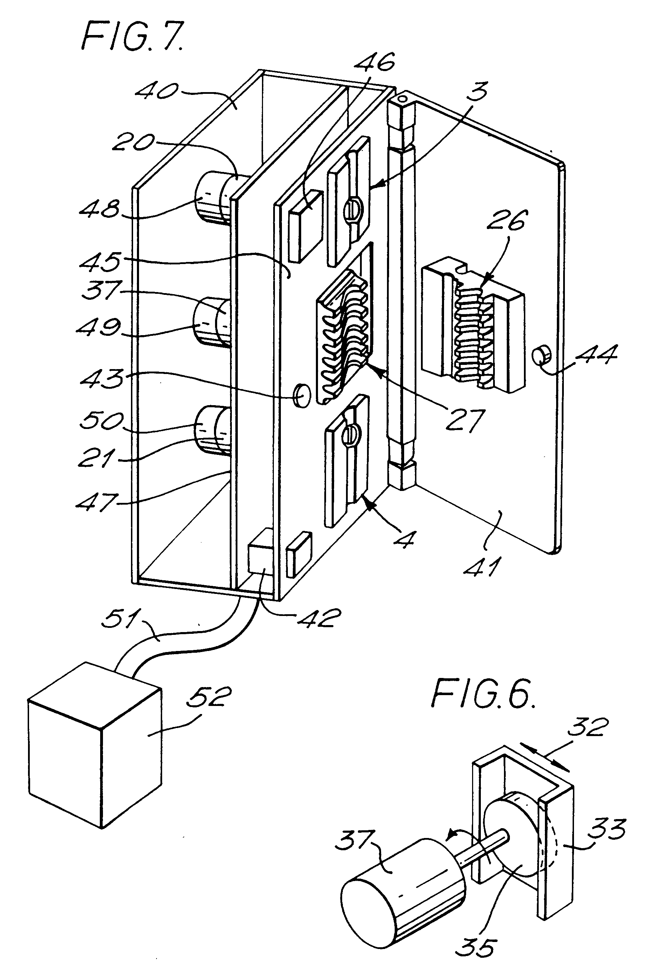

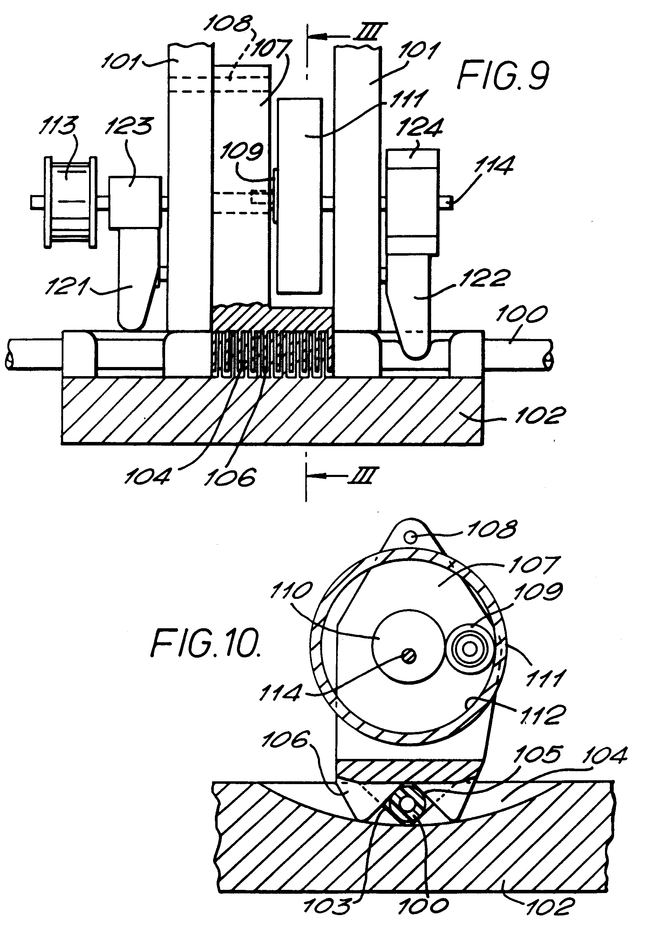

The member 27 is generally similar to the member 26 (as reflected by the use of like reference numbers for like parts) except that it is relatively inverted with, as best seen from Figure 4(a), the ridges 28 of one interdigitated with the ridges 28 of the other. Whilst in Figure 4(a), for ease of illustration, a gap is shown between the two members 26 and 27, in practice the ridges 28 of each member 26 or 27 bear on the surfaces 31 between the ridges 28 of the other member 27 or 26. Whilst not shown in Figures 4 and 5, but as more fully described with reference to Figure 8 later, the two members 26 and 27 are spring biased one towards the other. The member 26 is arranged to be stationary during operation whilst member 27 is arranged to move in the manner of a shuttle, to and fro as represented by the double headed arrow 32 in Figure 4. The means by which such shuttle like movement is accomplished is illustrated in Figure 6 which shows a cam follower 33 which is attached to the plain surface 34 (Figure 4) of the member 27, that is to say the obverse face relative to the face formed in the shape of the ridges 28. The cam follower 34 is driven to and fro by an eccentrically mounted drive wheel 35 driven by a motor 37, also shown and referenced as such in Figure 2. The effect of one cycle of movement of the member 27 relative to the member 26 is best seen from Figure 5. In (a) of Figure 5 the position of member 27 relative to member 26 is such that the semi-circular portions of the recesses in the ridges 28 of the two members 26,27 form a passage which is of substantially circular cross-section through the squeezing device in which the tubing 1 passes with no or no significant distortion. The diameter of the passage of circular cross-section is 4mm with tubing of 4.1mm outside diameter so as to provide a degree of "nip" at all times when the tubing is in place. As the motor 37 is driven in intermittent fashion so it produces rotation in a series of steps which moves the member 27 in the direction of the arrow 38 (Figure 5(b)) so as to squeeze the tube 1 to produce a cross-section which is oval in shape and of reduced area. At the limit of movement of the member 27 in the direction of the arrow 38 (as determined by the action of the cam wheel 35 and cam follower 33 and as illustrated in Figure 5(b)) the tubing does not occlude. That is to say, squeezing ceases before the approaching sides of the increasingly elliptical tubing make contact. In fact, the action of the ridges 28 of the members 26 and 27 on the tubing 1 induces a rolling motion of the tubing 1 so that this is not continually flexed in zones that are narrow in extent.. As the limit of movement of the member 27 in the direction of the arrow 38 is reached the drive applied to the motor 37 is changed from a series of short pulses to one long pulse producing accelerated rotation of the wheel 35 and reverse movement of the member 27 by virtue of the action of the cam wheel 35 and cam follower 33 with a relatively rapid return of the member 27 to the start position shown in Figure 5(a). This cycle repeats continuously whilst the pumping device is energised.

As the member 27 is moved shuttle-like as described above, so the controllable restrictive devices 3 and 4 are operated as already described and fluid is passed in a controlled fashion through the pumping device from input to output.

The precise timing of the shuttle-like movements of the member 27 relative to the member 26 and the operation of the controllably restrictive devices 3 and 4 may be seen from the following table. This is for a typical case with standard PVC tubing of 4mm outside diameter of which 35mm in length lies within the deforming means 2, using typical miniature d.c. electric motors for drive and to give a flow rate of 100cc's per hour.

| ACTION | ELAPSED TIME FROM START POSITION |
|---|---|
| CLOSE INLET | 0 |
| OPEN OUTLET | 100 |
| START SHUTTLE MOVEMENT OF MEMBER 27 IN DIRECTION OF ARROW 38 (FIGURE 5) | 200 |
| CLOSE OUTLET | 3000 |
| OPEN INLET | 3100 |
| START REVERSE MOVEMENT OF MEMBER 27 | 3200 |
| RETURNED TO START POSITION AND REPEAT | 3500 |

The time given in respect of each operation is in milliseconds from the "start" position. To move the member 27 in the direction of arrow 8 from the "start" position shown in Figure 5(a) to the limit of movement position shown in Figure 5(b), in a period of 3000 milliseconds, motor 37 is driven in discrete steps under electronic control (as known per se) to give a smooth flow of liquid.

A somewhat schematic perspective view of the complete pumping device is shown in Figure 8. All of the mechanical components, together with motors 20,21 and 37 and associated encoders controlling the motion of each are contained within a housing 40 shown with its outer casing removed. Housing 40 has a lid or door 41, shown opened.

Opening of the door 41 is controlled by means of a suitable latch, the details of which are not shown, operated by a push button 42 which extends through the casing when fitted. A microswitch arrangement of which the actuator button is represented at 43 is operated by a push rod 44 extending from the door 41 is arranged to deactivate the pumping device as the door 41 is opened, as will be described in more detail later.

The relatively stationary member 26 of the deforming means 2 as illustrated in Figure 4 is, as shown, carried by the door.

The mounting of the member 26 on the door 41 is such as to permit limited movement of the member away from the door and a spring (not shown in Figure 7) between the door and the member urges the latter towards the interior of the housing 40 (when the door is shut).

Within the housing 40 is a front panel 45 which carries the movable member 27 of the deforming means 2 (as described with reference to Figure 4) together with the controllable valve means 3,4 (as described with reference to Figure 3) arranged as described with reference to Figure 2. The front panel 45 also carries the aforementioned actuator button 43 of the microswitch and an operator-controlled push button switch 46 provided to command rotation of the rotary members 12,13 of the controllable restrictive means 3,4 to positions beyond their "start" positions (and against one stop) to enable the tubing 1 to be removed from the channels 8,9 in guides 6,7 as already described with reference to Figure 3.

Behind the front panel 45 is a printed circuit board 47 which carries the three drive motors 20,21 and 37 and their associated encoders, represented at 48,49,50.

The encoders 48,49,50 produce output signals indicative of the position of or the extent to which its associated motor has driven the respective member (rotary member of a controllable valve means or movable member of the squeezing device). Whilst each of motors 20,37 and 21 has an encoder 48,49 and 50 associated with it in this embodiment, in other embodiments the arrangement may be simplified (and cost saved) by providing only encoder 49 associated with shuttle motor 37 from which all necessary timing signals may be derived.

A twenty-way ribbon cable 51 connects the printed circuit board 47 to a remote microprocessor-based control unit 52 (also represented in Figure 2) containing a microprocessor and associated control electronics which is provided to control the movements of the motors 20,21 and 37 utilising the position indicative signals produced by the encoders 48,49,50 as reference signals. The cable 51 also carries to the microprocessor control unit 52 signals from the microswitch operated by actuator button 43 indicative of "door open" or "door shut" and signals from operator controlled push button switch 46.

The mounting of the relatively stationary member 26 of the deforming means in the door 41 and the mounting of the movable member 27 on the front panel 45 is shown in detail in Figure 8 which is a horizontal section through the relevant parts. Referring to Figure 8 the door 41 is hinged at 53. A recessed guide 54 extending inwardly from the inside of the door 41 holds the stationary member 26 of the deforming means captive whilst permitting limited movement towards and away from the door 41. A mounting block 55 on the inside of the door 41 and between the door 41 at the member 26 is recessed at 56. Recess 56 houses a coil spring 57 which extends into an aligned recess 58 in non-ridged (obverse) face of member 26. Spring 57 urges member 26 away from the door and thus into contact with the movable member 27 of the deforming means as previously described with reference to Figure 4.

The design of the microprocessor control unit 5 will be readily apparent to those skilled in the art from the following description of the sequence of operations of the pumping device described with reference to Figures 2 to 8.

Assuming that the door 41 is shut and the pumping apparatus is operating normally, opening the door 41 causes the microswitch actuated by actuator button 43 to send a "door open" indicative signal to the microprocessor control unit 52. Upon receipt, control unit 52 causes the outlet valve means 4 to close off the outlet and the inlet valve means 3 to close off the inlet. The moving member 27 of the deforming means 2 is returned to its position of minimum deformation, as illustrated in Figure 5(a). The pumping apparatus is now in a passive state, with the tubing captive in the channels 8,9 by virtue of the overhangs such as 17 in the case of rotary member 12, covering the channels.

It should now be assumed that the operator wishes to change the tubing 1. Normally the operator firstly closes a clamp (such as a standard roller clamp) fitted to the tubing 1, e.g. beyond the outlet. A label may conveniently be attached adjacent to the push button 46 to remind the operator to fit the clamp. Push button 46 is now operated and responsive to the signal thus generated control unit 52 causes the rotary members 12,13 to be rotated in a direction to release pressure on the tubing 1 (clockwise as viewed in Figure 2) beyond their normal "start" positions and against the stops provided to limit movement in that direction of rotation. As has already been described with reference to Figures 2 and 3, in this position the profiled surfaces of the projections (i.e. such as surface 16 of projection 14 of rotary member 12) are, broadly speaking, aligned with the channels 8,9 which are thus uncovered by the overhangs (e.g. overhang 17). Because the stationary member 26 has already been swung away from the moving member 27 of the deforming means 2 by the opening of the door 41, the tubing 1 may be removed.

Having discarded the tubing 1 and inserted a replacement, door 41 is shut. The microswitch thus operated by actuator button 43 signals again to the control unit to indicate "door shut". Responsive to this, control unit 52 causes outlet valve 4 to close off the outlet whilst inlet valve 3 remains in a condition in which the inlet is open (or is rotated to its "start" position). Moving member 27 of the deforming means 2 is driven to its extreme position of movement in a direction deforming the tube 1 and is then indexed back to its predetermined "start" position. At each extreme the positional indications provided by encoder 49 are noted by the microprocessor and serve to set up the index for subsequent operation. In addition to setting the device, this action also charges the length of tubing with liquid in through the opened inlet. The sequence of operation already described with reference to the table provided now commences with the closing of the inlet by inlet valve means 3.

Whilst the pumping apparatus described above is controlled by a microprocessor control unit, and this is preferred, the required timing and drives may be provided in other embodiments by discrete electrical components or indeed by mechanical means such as cam shafts and cam followers which are interconnected to operate in synchronisation. An example of such apparatus as last-mentioned, whilst not now preferred, will be described with reference to Figures 9 and 10.

Referring to Figures 9 and 10, 101 represents a fixed frame to which is adhered a base or anvil plate 102. The base plate will be separable from the frame 101, for example by being hinged, so that it may move away from the frame 101, for example swinging in gate fashion, so as to enable free access to be had to a V-shaped groove 103 formed in the baseplate 102 and defined by a plurality of ridges or blades 104 which may be integral with or fixed to the baseplate. However, whether or not it is hinged, in the operative condition of the apparatus, the baseplate 102 will be positioned as shown in Figures 9 and 10.

100 represents a length of hollow tubing, of plastics material such as p.v.c., laid in the V-shaped groeve 103 defined in the array of blades 104 of the baseplate 102. The tube length 100 is also located in a second groove 105 of opposed V-shape which groove 105 is defined by an array of blades 106 formed on an armature or shuttle 107 pivotally mounted by a pin or the like 108 on the frame 101. The blades 106 intermesh with the blades 104, as is best seen in Figure 10.

Pivotting on pin 108, the armature or shuttle 107 is moved back and forth in reciprocating arcuate movements of short stroke, by virtue of carrying a roller 109 engaging firstly against a cam 110 and secondly behind the circular rim 112, both the cam and the rim being concentric with one another and being fast on a wheel 111. The wheel 111 is fixed on a shaft 114 which is supported for rotation on frame 101, the shaft 114 being rotated by driven pulley 113. The cam 110 and the rim 112 are eccentric relative to the rotational axis of the shaft 114.

A motor whose operational speed is accurately adjustable, is employed the drive the pulley 113 through a cogged drive belt. In this way, the rate of shuttle movement and hence the rate of pumping can be accurately set. Normally these rates are set during assembly and are not routinely adjustable.

As the armature 107 swings to the left, in Figure 10, the righthand side of the V-shaped groove 105 defined by the blades 106 moves to the left and towards the lefthand side of V-shaped groove 103 defined by the blades 104. Conversely, as the armature swings to the right, in Figure 10, the lefthand side of V-shaped groove defined by the blades 106 moves to the right and towards the righthand side of the V-shaped groove defined by the blades 104. In this way the tubing confined in the space bounded by the opposed V-shaped grooves, is alternately squeezed from two different directions. The stroke of the swinging movement of the blades 106 is controlled to be such that the tubing is deformed, but not occluded.

If these two directions are substantially normal to one another, and if the tubing 100 is a relatively close fit in the space defined by the opposed V-shaped grooves 103 and 105, it will be appreciated that the configuration of the cross-section of the tubing is continually and positively controlled as it is squeezed alternately back and forth to adopt one or other of two elliptical cross-sectional configurations. Such control will ensure that during the transition from one elliptical shape to the other and back again there will be an intermediate stage during each transition when the tubing again adopts its shape when undeformed, that is to say it returns to a circular cross-section. The stroke of the armature 107, as it is reciprocated pivotally back and forth, is controlled, by the roller 109 engaging both the cam 110 and the wheel rim 112 as the wheel 111 rotates, such that the tubing never completely closes. Finally, the location of the tubing as a close fit in the intermeshing teeth defining the grooves will ensure that the tubing rolls or twists, rotating about its axis as it is successively deformed from, and restored to, its original shape, by the changing cross-section defined by the intermeshing teeth.

The opposing grooves 103 and 105 are, as stated above, respectively formed in two sets of intermeshing blades.

As will be appreciated whilst in the arrangement described with reference to Figures 9 and 10 the grooves defined by the blades are V-shaped, in fact shapes corresponding to the apertures in the ridges of the two members forming the squeezing device shown in Figure 4 could be applied here also. In addition the exemplary dimensions given for the intermeshing blades of the embodiment described with reference to Figures 9 and 10 may be applied to the ridges of the squeezing device described with reference to Figure 4.

In all of the embodiments described above if desired provision may readily be made whereby the flow rate may be altered during service, e.g. by adjustment to the cycle time.

It will also be appreciated that whilst a pumping device and apparatus in accordance with the present invention are primarily intended for medical applications, as previously mentioned, such apparatus and devices may find application in other fields.

## Claims

1. Pumping apparatus comprising a length of tubing (1) for providing passage of liquid through the apparatus, first and second members (26,27) defining a chamber receiving the tubing (1), the members (26,27) being relatively reciprocable to cause alternate deformation and reformation of the tubing (1) in a pumping action wherein the volume of the tubing (1) is reduced when it is deformed, the apparatus including means for urging the tubing (1) to its reformed state in which the normal cross-sectional shape of the tubing (1) is restored,
characterised in that, in a first relative position of the members (26,27), the chamber is substantially cylindrical, whereby the members (26,27) urge the tubing (1) to its reformed state, the members (26,27) being relatively movable in a direction radial of the chamber and tubing (1) to a second relative position, in which the chamber is elongate to cause deformation of the tubing (1), and controllable valve means (3,4) are provided upstream and downstream of the members to control the flow of liquid pumped through the tubing (1).

2. A pumping device for use in the apparatus of Claim 1, the device comprising first and second members (26,27) defining a chamber for receiving the tubing (1), the members (26,27) being relatively reciprocable, so as in use to cause alternate deformation and reformation of the tubing (1) in a pumping action wherein the volume of the tubing (1) is reduced when it is deformed, the device including means for urging deformed tubing (1) received in the chamber to its reformed state in which its normal cross-sectional shape is restored,
the device being characterised in that, in a first relative position of the members (26,27), the chamber is substantially cylindrical for urging tubing (1) in the chamber to its reformed state, the members (26,27) being relatively movable in a direction radial of the chamber to a second relative position, in which the chamber is elongate for causing deformation of tubing (1) received in the chamber.

3. A pumping apparatus as in Claim 1, wherein controllable valve means are positioned upstream and downstream of said members (26,27) and the apparatus comprises control means (52) operable to control the reciprocal movement of said members (26,27) and the operation of said valve means such that when said tubing is being deformed by said members (26,27), the valve means (3) on the upstream side restricts fluid flow through said tubing (1), whilst the valve means (4) on the downstream side permits increased liquid flow and when said tubing is being urged to its reformed state, the valve means (4) on the downstream side restricts fluid flow through the tubing (1) whilst the valve means (3) on the upstream side permits increased liquid flow.

4. Apparatus as claimed in Claim 1 or 3 wherein the valve means (3,4) are such that when fully operated, flow is stopped or permitted, as the case may be.

5. Apparatus as in Claim 1, 3 or 4, or a device as claimed in Claim 2, wherein the members are constructed to deform said tubing (1) in a non-occlusive manner.

6. Apparatus as in any one of Claims 1 and 3 to 5, or a device as claimed in any of Claims 2 and 4 to 5, wherein the members (26,27) are movable from said first position in a direction that is opposite to said direction such that the tubing is urged beyond restoration of its normal cross-sectional shape to cause further local deformation of the cross-sectional shape of said tubing (1).

7. Apparatus as in any one of Claims 1 and 3 to 6, or a device as claimed in any of Claims 2 and 4 to 6 wherein said members each has a valley, one of said two members being inverted relative to the other with their valleys together to form said chamber.

8. Apparatus or a device as claimed in Claim 7, wherein each member has a series of transverse blades or ridges (28) shaped to provide said valley, the ridges (28) of one member being interdigitated with the ridges (28) of the other member.

9. Apparatus or a device as claimed in Claim 8 and wherein each ridge (28) has a recess which is generally semi-circular to one side (29) and of progressively decreasing depth to the other side (30) until the full height of the ridge (28) is reached.

10. Apparatus or a device as claimed in Claim 9 and wherein all of the ridges (28) of one member (26,27) are substantially identical, with the generally semi-circular portions of their apertures to the same side (29).

11. Apparatus or a device as claimed in any of Claims 8 to 10 and wherein, viewed in the axial direction of said chamber, all of the ridges (28) of one member (26,27) appear superimposed.

12. Apparatus or a device as claimed in any one of Claims 9, 10 and 11 and wherein the generally semi-circular portions of the apertures in the ridges (28) of one member are to one side and the generally semi-circular portions of the apertures in the ridges (28) of the relatively inverted member are to the other side, as viewed in the axial direction of said chamber.

13. Apparatus or a device as claimed in any one of Claims 9 to 12, wherein the generally semi-circular portions of the ridges (28) of the two members together form said substantially cylindrical chamber when the members (26,27) are in the first position, the cylinder having a diameter closely similar to that of said tubing (1).

14. Apparatus or a device as claimed in Claim 13 and wherein said chamber diameter slightly less than that of said tubing (1) whereby gently to nip said tubing (1).

15. Apparatus or a device as claimed in any of Claims 8 to 14 and wherein members are movable such that the ridges (28) of one member bear on the surfaces (31) between the ridges (28) of the other member.

16. Apparatus or a device as claimed in any preceding claim wherein said members are biassed one towards the other by resilient means (57).

17. Apparatus or a device as claimed in Claim 16 and wherein said resilient means is a spring (57).

18. Apparatus as in any one of Claims 1 and 3 to 17 or a device as claimed in any one of Claims 2 or 5 to 17, wherein one of said members (26) is arranged to be stationary during operation whilst the other member (27) is movable in the manner of a shuttle.

19. Apparatus as claimed in Claim 18 comprising a housing (40) with a closure (41), such as a door or lid and wherein the member (26) which is arranged to be stationary during operation is carried by said closure (41), and wherein said closure (41) is openable to release said tubing (1) from said chamber.

20. Apparatus as claimed in Claim 19 and wherein the member (26) which is arranged to be stationary during operation has limited freedom to move, independent of said closure (41), towards and away from said other member (27), biassing means (57) being provided between it and said closure (41) tending to urge it towards said other member (27).

21. Apparatus as claimed in any of Claims 18 to 20 and wherein said member (27) which is arranged to move in the manner of a shuttle is drivable via an eccentric (35) by an electric motor (37) which is controllable to move, in operation, in a series of discrete steps producing incremental steps of said member in said direction producing deformation of said tubing (1).

22. Apparatus as claimed in Claim 21 and wherein said motor is operable to return the member (27) movable in the manner of a shuttle in a direction opposite to said direction in one relatively rapid movement.

23. Apparatus as claimed in any of Claims 1 and 3 to 22, wherein said or each valve means (3,4) comprises a guide member (6) having a channel therethrough for said tubing (1) and, within said channel (8,9), a rotary member (12,13) having an off-centre projection (14) extending generally parallel to the axis of rotation of said rotary member (12,13) and having one face (16) against which said tubing (1) lies, the face (16) defining in part said channel (8,9), partial rotation of said rotary member (12,13) causing said tubing (1) to be occluded by the resultant action of said face (16) upon said tubing (1).

24. Apparatus as claimed in Claim 23 and wherein said face (16) of said projection (14) is partially recessed with a profiled surface, said rotary member (12,13) being partially rotatable to a "start" position to produce a overhang (17) which provides a closure over the channel (8,9) in said guide member (6) capturing said tubing (1) therein with said profiled surface bearing on the tubing (1).

25. Apparatus as claimed in Claim 23 or 24 and wherein stop means (18,19) are provided for engagement with each rotary member (12,13), each rotary member being rotatable in one direction to a position in which the respective rotary member engages the stop means (19) to limit movement of the rotary member and to position the rotary member such that the face (16) of the rotary member is so aligned with the tubing (1) as to permit said tubing (1) to be removed from said channel (8,9) of the rotary member, and in the other direction of rotation to a second position beyond that at which said tubing (1) is occluded.

26. Apparatus as claimed in Claim 25 and wherein each of said rotary members (12,13) is rotatable such that in normal operation said second position is not reached.

27. Apparatus as claimed in Claim 25 or 26 and wherein the stop means comprises an arcuate slot or recess (18) in each rotary member, each slot or recess being coaxial with the axis of rotation of the respective rotary member (12,13) and co-operable with a fixed pin or other abutment (19).

28. Apparatus as claimed in Claim 3, wherein the valve means (3,4) are operable without, or substantially without, overlap between the periods at which each is permitting increased liquid flow.

29. Apparatus as claimed in Claim 25 when dependent from Claim 3 wherein said control means (52) is operable to cause rotation of the rotary members (12,13) to the first position in which the face (16) of each rotary member (12,13) is so aligned with the tubing, and movement of the deforming members (26,27) to the first position, so as to permit the tubing (1) to be removed from said channels (8,9) and chamber.

30. Apparatus as claimed in Claim 19 when dependent upon Claim 3 and wherein said control means (52) is operable to cause both said valve means (3,4) to restrict the flow of liquid in the tubing (1) when said closure (41) is opened.

31. Apparatus as claimed in Claim 30 and wherein said control means (52) is operable on opening the closure (41), to cause the movable member (27) to move to said first relative position after the valve means (3,4) restrict said flow.

32. Apparatus as claimed in Claim 31 and wherein the control means (52) is operable on closing the closure (41) to cause the movable member (27) to move to said second relative position, with the downstream valve means restricting liquid flow and the upstream valve means permitting increased liquid flow and thereafter to move with both valve means (3,4) remaining in the conditions just mentioned to a predetermined start position, whereafter the control means (52) is operable to cause the upstream means to restrict liquid flow and the downstream valve means to permit increased liquid flow, and to cause the operation cycle of said valve means (3,4) and reciprocal movement of said members (26,27) to commence.

33. Apparatus as claimed in Claim 31 comprising operator controllable means (46) accessible to an operator only when said closure (41) is open and operable after the movable member (27) has moved to said first relative position on opening said closure (41).

34. Apparatus as claimed in Claim 23 when dependent from Claim 18, wherein each rotary member (12,13) and the moving member (27) are drivable by dedicated electric motors (20,21,37) which are controlled by a microprocessor (52).

35. Apparatus as claimed in Claim 34 and wherein at least the motor (37) provided for driving the movable member (27) has associated therewith an encoder (49) which produces an output signal indicative of the position of or extent to which the member (27) driven by that motor (37) has moved, means (51) being provided for passing the signals thus produced to said microprocessor (52) for use as reference signals in the timing of the generation of motor control signals by said microprocessor (52).

## Patentansprüche

1. Pumpvorrichtung, die ein Stück Schlauch (1), um einen Flüssigkeitsdurchlaß durch die Vorrichtung zu bilden, und ein erstes und ein zweites Element (26, 27) aufweist, die eine den Schlauch (1) aufnehmende Kammer bilden, wobei die Elemente (26, 27) relativ zueinander hin- und herbewegbar sind, um ein abwechselndes Verformen und Rückformen des Schlauchs (1) in einem Pumpvorgang zu bewirken, wobei das Volumen des Schlauchs (1) verringert wird, wenn er verformt wird, und wobei die Vorrichtung eine Einrichtung aufweist, um den Schlauch (1) in seinen rückgeformten Zustand zu drängen, in dem die normale Querschnittsgestalt des Schlauchs (1) wiederhergestellt ist,
dadurch gekennzeichnet,
daß in einer ersten relativen Position der Elemente (26, 27) die Kammer im wesentlichen zylindrisch ist, so daß die Elemente (26, 27) den Schlauch (1) in seinen rückgeformten Zustand drängen, wobei die Elemente (26, 27) in einer Richtung radial zu der Kammer und dem Schlauch (1) in eine zweite relative Position relativ zueinander bewegbar sind, in der die Kammer langgestreckt ist, um eine Verformung des Schlauchs (1) zu bewirken, und wobei steuerbare Ventilelemente (3, 4) an der Aufstromseite und der Abstromseite der Elemente vorgesehen sind, um den Durchfluß von durch den Schlauch (1) gepumpter Flüssigkeit zu steuern.

2. Pumpeinrichtung zur Verwendung in der Vorrichtung nach Anspruch 1, wobei die Einrichtung ein erstes und ein zweites Element (26, 27) aufweist, die eine Kammer bilden, um den Schlauch (1) aufzunehmen, die Elemente (26, 27) relativ zueinander hin- und herbewegbar sind, um im Gebrauch ein abwechselndes Verformen und Rückformen des Schlauchs in einem Pumpvorgang zu bewirken, wobei das Volumen des Schlauchs (1) verringert wird, wenn er verformt wird, wobei die Einrichtung Mittel aufweist, um den in der Kammer aufgenommenen verformten Schlauch (1) in seinen rückgeformten Zustand zu drängen, in dem seine normale Querschnittsgestalt wiederhergestellt ist,
wobei die Einrichtung dadurch gekennzeichnet ist,
daß in einer ersten relativen Position der Elemente (26, 27) die Kammer im wesentlichen zylindrisch ist, um den Schlauch (1) in der Kammer in seinen rückgeformten Zustand zu drängen, wobei die Elemente (26, 27) in einer Richtung radial zu der Kammer in eine zweite relative Position relativ zueinander bewegbar sind, in der die Kammer langgestreckt ist, um eine Verformung des in der Kammer aufgenommenen Schlauchs (1) zu bewirken.

3. Pumpvorrichtung nach Anspruch 1,
wobei die steuerbaren Ventilelemente an der Aufstromseite und der Abstromseite der Elemente (26, 27) positioniert sind und die Vorrichtung eine Steuereinrichtung (52) aufweist, die betätigbar ist, um die Hin- und Herbewegung der Elemente (26, 27) und den Betrieb der Ventilelemente derart zu steuern, daß dann, wenn der Schlauch von den Elementen (26, 27) verformt wird, das Ventilelement (3) an der Aufstromseite einen Fluiddurchfluß durch den Schlauch (1) begrenzt, während gleichzeitig das Ventilelement (4) an der Abstromseite einen erhöhten Flüssigkeitsdurchfluß zuläßt und, wenn der Schlauch in seinen rückgeformten Zustand gedrängt wird, das Ventilelement (4) an der Abstromseite den Fluiddurchfluß durch den Schlauch (1) begrenzt, während gleichzeitig das Ventilelement (3) an der Aufstromseite einen erhöhten Flüssigkeitsdurchfluß zuläßt.

4. Vorrichtung nach Anspruch 1 oder 3,
wobei die Ventilelemente (3, 4) derart ausgebildet sind, daß dann, wenn sie vollständig betätigt sind, der Durchfluß blockiert bzw. zugelassen wird.

5. Vorrichtung nach Anspruch 1, 3 oder 4 oder Einrichtung nach Anspruch 2,
wobei die Elemente so ausgebildet sind, daß sie den Schlauch (1) auf eine nichtverschließiende Weise verformen.

6. Vorrichtung nach einem der Ansprüche 1 und 3 bis 5 oder Einrichtung nach einem der Ansprüche 2 und 4 bis 5,
wobei die Elemente (26, 27) aus der ersten Position in einer Richtung bewegbar sind, die zu der genannten Richtung entgegengesetzt ist, so daß der Schlauch über eine Rückstellung in seine normale Querschnittsgestalt hinaus beaufschlagt wird, um weiteres lokales Verformen der Querschnittsgestalt des Schlauchs (1) zu bewirken.

7. Vorrichtung nach einem der Ansprüche 1 und 3 bis 6 oder Einrichtung nach einem der Ansprüche 2 und 4 bis 6,
wobei jedes der Elemente eine Vertiefung hat, wobei das eine der beiden Elemente relativ zu dem anderen umgekehrt ist, so daß ihre Vertiefungen zusammenkommen, um die Kammer bilden.

8. Vorrichtung oder Einrichtung nach Anspruch 7,
wobei jedes Element eine Reihe von quer verlaufenden Stegen oder Rippen (28) hat, die so geformt sind, daß sie die Vertiefung bilden, wobei die Rippen (28) eines Elements mit den Rippen (28) des anderen Elements fingerartig ineinandergreifen.

9. Vorrichtung oder Einrichtung nach Anspruch 8,
wobei jede Rippe (28) eine Ausnehmung hat, die zu der einen Seite (29) im allgemeinen halbkreisförmig ist und zu der anderen Seite (30) eine progressiv abnehmende Tiefe hat, bis die volle Höhe der Rippe (28) erreicht ist.

10. Vorrichtung oder Einrichtung nach Anspruch 9,
wobei alle Rippen (28) eines Elements (26, 27) im wesentlichen identisch sind, wobei die im allgemeinen halbkreisförmigen Bereiche ihrer Öffnungen zu der gleichen Seite (29) weisen.

11. Vorrichtung oder Einrichtung nach einem der Ansprüche 8 bis 10,
wobei, in der Axialrichtung der Kammer gesehen, alle Rippen (28) eines Elements (26, 27) übereinanderliegend erscheinen.

12. Vorrichtung oder Einrichtung nach einem der Ansprüche 9, 10 und 11,
wobei die im allgemeinen halbkreisförmigen Bereiche der Öffnungen in den Rippen (28) eines Elements zu der einen Seite weisen und die im allgemeinen halbkreisförmigen Bereiche der Öffnungen in den Rippen (28) des relativ dazu umgekehrten Elements zu der anderen Seite weisen, gesehen in der Axialrichtung der Kammer.

13. Vorrichtung oder Einrichtung nach einem der Ansprüche 9 bis 12,
wobei die im allgemeinen halbkreisförmigen Bereiche der Rippen (28) der beiden Elemente gemeinsam die im wesentlichen zylindrische Kammer bilden, wenn die Elemente (26, 27) in der ersten Position sind, wobei der Zylinder einen Durchmesser hat, der dem des Schlauchs (1) weitgehend ähnlich ist.

14. Vorrichtung oder Einrichtung nach Anspruch 13,
wobei der Kammerdurchmesser geringfügig kleiner als der des Schlauchs (1) ist, um den Schlauch (1) dadurch sanft einzuklemmen.

15. Vorrichtung oder Einrichtung nach einem der Ansprüche 8 bis 14,
wobei die Elemente derart bewegbar sind, daß die Rippen (28) des einen Elements an den Oberflächen (31) zwischen den Rippen (28) des anderen Elements anliegen.

16. Vorrichtung oder Einrichtung nach einem der vorhergehenden Ansprüche,
wobei die Elemente durch eine federnde Einrichtung (57) aufeinander zu vorgespannt sind.

17. Vorrichtung oder Einrichtung nach Anspruch 16,
wobei die federnde Einrichtung eine Feder (57) ist.

18. Vorrichtung nach einem der Ansprüche 1 und 3 bis 17 oder Einrichtung nach einem der Ansprüche 2 oder 5 bis 17,
wobei das eine der Elemente (26) so angeordnet ist, daß es im Betrieb ortsfest ist, während gleichzeitig das andere Element (27) nach Art eines hin- und hergehenden Körpers bewegbar ist.

19. Vorrichtung nach Anspruch 18,
die ein Gehäuse (40) mit einem Verschluß (41), wie etwa einer Tür oder einem Deckel aufweist und wobei das Element (26), das so angeordnet ist, daß es im Betrieb ortsfest ist, von dem Verschluß (41) getragen ist und wobei der Verschluß (41) öffnungsfähig ist, um den Schlauch (1) aus der Kammer freizugeben.

20. Vorrichtung nach Anspruch 19,
wobei das Element (26), das so angeordnet ist, daß es im Betrieb ortsfest ist, unabhängig von dem Verschluß (41) eine begrenzte Freiheit hat, sich auf das andere Element (27) zu und davon weg zu bewegen, wobei die Vorspanneinrichtung (57), die zwischen ihm und dem Verschluß (41) vorgesehen ist, dazu tendiert, es auf das andere Element (27) zu zu drängen.

21. Vorrichtung nach einem der Ansprüche 18 bis 20,
wobei das Element (27), das so angeordnet ist, daß es sich nach Art eines hin- und hergehenden Körpers bewegt, über einen Exzenter (35) von einem Elektromotor (37) antreibbar ist, der steuerbar ist, um sich im Betrieb in einer Reihe von diskreten Schritten zu bewegen, die inkrementelle Schritte des Elements in der genannten Richtung erzeugen, die eine Verformung des Schlauchs (1) erzeugen.

22. Vorrichtung nach Anspruch 21,
wobei der Motor betätigbar ist, um das Element (27), das nach Art eines hin- und hergehenden Körpers bewegbar ist, in einer relativ schnellen Bewegung in einer Richtung zurückzuführen, die zu der genannten Richtung entgegengesetzt ist.

23. Vorrichtung nach einem der Ansprüche 1 und 3 bis 22,
wobei das oder jedes Ventilelement (3, 4) ein Führungselement (6) mit einem hindurchgehenden Kanal für den Schlauch (1) und in dem Kanal (8, 9) ein Drehelement (12, 13) aufweist, das einen außermittigen Vorsprung (14) hat, der im allgemeinen parallel zu der Drehachse des Drehelements (12, 13) verläuft und eine Fläche (16) hat, an der der Schlauch (1) anliegt, wobei die Fläche (16) den Kanal (8, 9) teilweise bildet, wobei eine teilweise Drehung des Drehelements (12, 13) bewirkt, daß der Schlauch (1) durch die resultierende Einwirkung der Fläche (16) auf den Schlauch (1) verschlossen wird.

24. Vorrichtung nach Anspruch 23,
wobei die Fläche (16) des Vorsprungs (14) mit einer profilierten Oberfläche teilweise mit Aussparungen versehen ist, wobei das Drehelement (12, 13) in eine "Start"-Position teilweise drehbar ist, um einen Überhang (17) zu erzeugen, der einen Verschluß über dem Kanal (8, 9) in dem Führungselement (6) bildet, der den Schlauch (1) darin festlegt, wobei die profilierte Oberfläche an dem Schlauch (1) anliegt.

25. Vorrichtung nach Anspruch 23 oder 24,
wobei Anschlageinrichtungen (18, 19) für den Eingriff mit jedem Drehelement (12, 13) vorgesehen sind, wobei jedes Drehelement in der einen Richtung in eine Position drehbar ist, in der das jeweilige Drehelement mit der Anschlageinrichtung (19) in Eingriff gelangt, um die Bewegung des Drehelements zu begrenzen und das Drehelement derart zu positionieren, daß die Fläche (16) des Drehelements mit dem Schlauch (1) so ausgefluchtet ist, daß das Entfernen des Schlauchs (1) aus dem Kanal (8, 9) des Drehelements heraus möglich ist, und in der anderen Drehrichtung in eine zweite Position über diejenige hinaus drehbar ist, in der der Schlauch (1) verschlossen wird.

26. Vorrichtung nach Anspruch 25,
wobei jedes der Drehelemente (12, 13) derart drehbar ist, daß im Normalbetrieb die zweite Position nicht erreicht wird.

27. Vorrichtung nach Anspruch 25 oder 26,
wobei die Anschlageinrichtung einen bogenförmigen Schlitz oder eine bogenförmige Ausnehmung (18) in jedem Drehelement aufweist, wobei jeder Schlitz oder jede Ausnehmung mit der Drehachse des jeweiligen Drehelements (12, 13) koaxial ist und mit einem ortsfesten Stift oder sonstigen Anschlag (19) zusammenwirken kann.

28. Vorrichtung nach Anspruch 3,
wobei die Ventilelemente (3, 4) ohne oder im wesentlichen ohne Überlappung zwischen den Perioden betätigbar sind, in denen jedes einen erhöhten Flüssigkeitsdurchfluß zuläßt.

29. Vorrichtung nach Anspruch 25 in Abhängigkeit von Anspruch 3,
wobei die Steuereinrichtung (52) betätigbar ist, um eine Drehung der Drehelemente (12, 13) in die erste Position, in der die Fläche (16) jedes Drehelements (12, 13) mit dem Schlauch ausgefluchtet ist, und eine Bewegung der Verformungselemente (26, 27) in die erste Position zu bewirken, um das Entfernen des Schlauchs (1) aus den Kanälen (8, 9) und der Kammer zuzulassen.

30. Vorrichtung nach Anspruch 19 in Abhängigkeit von Anspruch 3,
wobei die Steuereinrichtung (52) betätigbar ist, um beide Ventilelemente (3, 4) dazu zu veranlassen, den Flüssigkeitsdurchfluß in dem Schlauch (1) zu begrenzen, wenn der Verschluß (41) geöffnet wird.

31. Vorrichtung nach Anspruch 30,
wobei die Steuereinrichtung (52) beim Öffnen des Verschlusses (41) betätigbar ist, um das bewegliche Element (27) dazu zu veranlassen, sich in die erste relative Position zu bewegen, wenn die Ventilelemente (3, 4) den Durchfluß begrenzen.

32. Vorrichtung nach Anspruch 31,
wobei die Steuereinrichtung (52) beim Schließen des Verschlusses (41) betätigbar ist, um das bewegliche Element (27) dazu zu veranlassen, sich in die zweite relative Position zu bewegen, wobei das abstromseitige Ventilelement den Flüssigkeitsdurchfluß begrenzt und das aufstromseitige Ventilelement einen erhöhten Flüssigkeitsdurchfluß zuläßt, und um sich danach in eine vorbestimmte Start-Position zu bewegen, wobei die beiden Ventilelemente (3, 4) in den eben genannten Zuständen verbleiben, woraufhin die Steuereinrichtung (52) betätigbar ist, um zu bewirken, daß das aufstromseitige Ventilelement den Flüssigkeitsdurchfluß begrenzt und das abstromseitige Ventilelement einen erhöhten Flüssigkeitsdurchfluß zuläßt, und um zu bewirken, daß der Arbeitszyklus der Ventilelemente (3, 4) und die Hin- und Herbewegung der Elemente (26, 27) beginnen.

33. Vorrichtung nach Anspruch 31,
die eine von einem Bediener steuerbare Einrichtung (46) aufweist, die für einen Bediener nur dann zugänglich ist, wenn der Verschluß (41) offen ist, und die erst betätigbar ist, nachdem sich das bewegliche Element (27) beim Öffnen des Verschlusses (41) in die erste relative Position bewegt hat.

34. Vorrichtung nach Anspruch 23 in Abhängigkeit von Anspruch 18,
wobei jedes Drehelement (12, 13) und das bewegliche Element (27) von zugeordneten Elektromotoren (20, 21, 37) antreibbar sind, die von einem Mikroprozessor (52) gesteuert werden.

35. Vorrichtung nach Anspruch 34,
wobei zumindest der Motor (37), der vorgesehen ist, um das bewegliche Element (27) anzutreiben, einen ihm zugeordneten Codierer (49) hat, der ein Ausgangssignal erzeugt, das die Position des von diesem Motor (37) angetriebenen Elements (27) oder das Ausmaß, in dem sich dieses bewegt hat, bezeichnet, wobei eine Einrichtung (51) vorgesehen ist, um die so erzeugten Signale dem Mikroprozessor (52) zuzuführen, damit diese als Referenzsignale bei der zeitlichen Steuerung der Erzeugung von Motorsteuersignalen durch den Mikroprozessor (52) verwendet werden.

## Revendications

1. Appareil de pompage comprenant une longueur de tube (1) pour assurer le passage du liquide à travers l'appareil, des premier et deuxième éléments (26, 27) définissant une chambre recevant le tube (1), les éléments (26, 27) pouvant se déplacer relativement d'un mouvement de va et vient pour provoquer des déformations et des remises en forme alternées du tube (1) suivant une action de pompage dans laquelle le volume du tube (1) est diminué quand il est déformé, l'appareil comprenant un moyen pour pousser le tube (1) jusqu'à sa forme initiale qui rétablit la forme normale de la section du tube 1,
caractérisé en ce que, dans une première position relative des éléments (26, 27), la chambre est essentiellement cylindrique, alors que les éléments (26, 27) poussent le tube (1) pour le remettre dans sa forme initiale, les éléments (26, 27) étant mobiles relativement dans une direction radiale de la chambre du tube (1), jusqu'à une deuxième position relative dans laquelle la chambre est allongée en provoquant la déformation du tube (1) et des vannes contrôlables (3, 4) étant disposées en amont et en aval des éléments pour commander le débit de liquide pompé à travers le tube (1).

2. Dispositif de pompage à utiliser dans l'appareil de la revendication 1, le dispositif comprenant un premier et un deuxième éléments (26, 27), définissant une chambre destinée à recevoir le tube (1), les éléments (26, 27) pouvant être animés d'un mouvement relatif de va et vient, de façon à être utilisés pour provoquer une déformation et une remise en forme alternées du tube (1) suivant une action de pompage dans laquelle le volume du tube (1) est diminué quand il est déformé, le dispositif comprenant un moyen pour pousser le tube déformé (1) placé dans la chambre, en le remettant dans sa forme initiale qui rétablit la forme normale de sa section,
le dispositif étant caractérisé en ce que, dans une première position relative des éléments (26, 27), la chambre est essentiellement cylindrique pour pousser le tube (1) dans la chambre jusqu'à sa forme initiale, les éléments (26, 27) étant mobiles relativement dans une direction radiale de la chambre vers une deuxième position relative dans laquelle la chambre est allongée pour provoquer la déformation du tube (1) placé dans la chambre.

3. Appareil de pompage selon la revendication 1, dans lequel des vannes contrôlables sont placées en amont et en aval desdits éléments (26, 27) et l'appareil comprend un moyen de commande (52) pouvant être actionné pour commander le mouvement de va et vient desdits éléments (26, 27) et le fonctionnement desdites vannes, de façon que, lorsque ledit tube est déformé par lesdits éléments (26, 27), la vanne (3) sur le côté amont diminue le débit de liquide à travers ledit tube (1), tandis que la vanne (4) du côté aval permet un débit de liquide augmenté et, quand ledit tube est ramené à sa forme initiale, la vanne (4) du côté aval diminue le débit du liquide à travers le tube (1), tandis que la vanne (3) du côté amont permet un débit de liquide augmenté.

4. Appareil selon la revendication 1 ou 3, dans lequel les vannes (3, 4) sont telles que, quand elles sont complètement actionnées, le débit est interrompu ou rendu possible suivant le cas.

5. Appareil selon la revendication 1, 3 ou 4 ou dispositif selon la revendication 2, dans lequel les éléments sont constitués de manière à déformer ledit tube (1) sans le fermer.

6. Appareil selon l'une quelconque des revendications 1 et 3 à 5 ou dispositif selon l'une quelconque des revendications 2 et 4 à 5, dans lesquels les éléments (26, 27) peuvent se déplacer depuis ladite première position dans une direction opposée à ladite direction telle que le tube soit poussé au-delà du rétablissement de la forme normale de sa section, en provoquant une déformation locale supplémentaire de la forme de la section dudit tube (1).

7. Appareil selon l'une quelconque des revendications 1 et 3 à 6, ou dispositif selon l'une quelconque des revendications 2 et 4 à 6, dans lesquels lesdits éléments ont chacun une vallée, l'un desdits éléments étant inversé par rapport à l'autre et leurs vallées formant ensemble ladite chambre.

8. Appareil ou dispositif selon la revendication 7, dans lesquels chaque élément comporte une série de lames ou de crêtes transversales (28) profilées de manière à constituer ladite vallée, les crêtes (28) d'un élément étant interposées entre les crêtes (28) de l'autre élément.

9. Appareil ou dispositif selon la revendication 8, dans lesquels chaque crête (28) a un redan qui a une forme généralement circulaire sur un côté (29) et une profondeur diminuant progressivement de l'autre côté (30) jusqu'à ce que la hauteur totale de la crête (28) soit atteinte.

10. Appareil ou dispositif selon la revendication 9, dans lesquels toutes les crêtes (28) d'un élément (26, 27) sont essentiellement identiques avec les parties généralement semi-circulaires de leurs ouvertures, du même côté 29.

11. Appareil ou dispositif selon l'une quelconque des revendications 8 à 10, dans lesquels, quand on regarde dans la direction axiale de ladite chambre, toutes les crêtes 28 d'un élément (26, 27) apparaissent en superposition.

12. Appareil ou dispositif selon l'une quelconque des revendications 9, 10 et 11, dans lesquels les parties généralement semi-circulaires des ouvertures dans les crêtes (28) d'un élément sont d'un côté et les parties généralement semi-circulaires des ouvertures dans les crêtes (28) de l'élément inversé relativement sont de l'autre côté quand on regarde dans la direction axiale de ladite chambre.

13. Appareil ou dispositif selon l'une quelconque des revendications 9 à 12, dans lesquels les parties généralement circulaires des crêtes (28) des deux éléments forment ensemble ladite chambre essentiellement cylindrique quand les éléments (26, 27) sont dans la première position, le cylindre ayant un diamètre très semblable à celui dudit tube (1).

14. Appareil ou dispositif selon la revendication 13 et dans lesquels ledit diamètre de la chambre est légèrement moindre que celui dudit tube (1), de manière à pincer légèrement ledit tube (1).

15. Appareil ou dispositif selon l'une quelconque des revendications 8 à 14, dans lesquels les éléments sont mobiles de façon que les crêtes (28) d'un élément appuient sur les surfaces (31) entre les crêtes (28) de l'autre élément.

16. Appareil ou dispositif selon l'une quelconque des revendications précédentes, dans lesquels lesdits éléments sont poussés l'un vers l'autre par un moyen élastique (57).

17. Appareil ou dispositif selon la revendication 16, dans lesquels ledit moyen élastique est un ressort (57).

18. Appareil selon l'une quelconque des revendications 1 et 3 à 17 ou dispositif selon l'une quelconque des revendications 2 ou 5 à 17, dans lesquels l'un desdits éléments (26) est disposé de manière à être fixe pendant le fonctionnement, alors que l'autre élément (27) est mobile à la manière d'une navette.

19. Appareil selon la revendication 18, comprenant un logement (40) avec une fermeture (41) telle qu'une porte ou un couvercle, dans lequel l'élément (26), qui est disposé pour être fixe pendant le fonctionnement, est supporté par ladite fermeture (41) et dans lequel ladite fermeture (41) peut être ouverte pour dégager ledit tube (1) de ladite chambre.

20. Appareil selon la revendication 19, dans lequel l'élément (26), qui est disposé pour être fixe pendant le fonctionnement, a une liberté de mouvement limitée indépendante de ladite fermeture (41) vers ledit autre élément (27) et avec écartement par rapport à celui-ci, un moyen de poussée (57) étant disposé entre celui-ci et ladite fermeture (41), de manière à le pousser vers l'autre élément (27).

21. Appareil selon l'une quelconque des revendications 18 à 20, dans lequel ledit élément (27), qui est disposé pour se déplacer à la manière d'une navette, peut être entraîné par l'intermédiaire d'un excentrique (35) au moyen d'un moteur électrique (37) qui est contrôlable, de manière à se déplacer en fonctionnement suivant une série d'étapes séparées, en produisant des déplacements successifs dudit élément dans une direction qui provoque la déformation dudit tube (1).

22. Appareil selon la revendication 21, dans lequel ledit moteur peut être actionné pour ramener l'élément (27) mobile à la manière d'une navette dans une direction opposée à ladite direction suivant un mouvement relativement rapide.

23. Appareil selon l'une quelconque des revendications 1 et 3 à 22, dans lequel chacune desdites vannes (3, 4) comporte un élément de guidage (6) ayant à travers celui-ci un passage pour ledit tube (1) et dans ledit passage (8, 9), un élément rotatif 12, 13 ayant une saillie excentré (14) s'étendant de manière généralement parallèle à l'axe de rotation dudit élément rotatif (12, 13) et ayant une face (16) contre laquelle repose ledit tube (1), la face (16) définissant partiellement ledit passage (8, 9), la rotation partielle dudit élément rotatif (12, 13) provoquant la fermeture dudit tube (1) sous l'action résultante de ladite face (16) agissant sur ledit tube (1).

24. Appareil selon la revendication 23, dans lequel ladite face (16) de ladite saillie (14) est partiellement en retrait avec une surface profilée, ledit élément rotatif (12, 13) pouvant tourner partiellement jusqu'à une position de démarrage en produisant un surplomb 17 qui assure une fermeture par-dessus le passage (8, 9) dans ledit élément de guidage (7), en maintenant ledit tube (1) dans celui-ci avec la surface profilée appuyant sur le tube (1).

25. Appareil selon la revendication 23 ou 24, dans lequel des butées (18, 19) sont prévues pour venir en contact avec chaque élément rotatif (12, 13), chaque élément rotatif pouvant tourner dans un sens jusqu'à une position dans laquelle l'élément rotatif correspondant est en contact avec la butée 19 pour limiter le déplacement de l'élément rotatif et pour positionner l'élément rotatif de façon que la face 16 de l'élément rotatif soit alignée avec le tube (1), de façon à permettre l'enlèvement du tube (1) dudit passage (8, 9) de l'élément rotatif et, dans l'autre sens de rotation, jusqu'à une deuxième position au-delà de laquelle ledit tube (1) est fermé.

26. Appareil selon la revendication 25 et dans lequel chacun desdits éléments rotatifs (12, 13) peut tourner de façon qu'en fonctionnement normal ladite deuxième position ne soit pas atteinte.

27. Appareil selon la revendication 25 ou 26 et dans lequel la butée comporte une fente ou un redan incurvé (18) dans chaque élément rotatif, chaque fente ou redan étant coaxial par rapport à l'axe de rotation de l'élément rotatif correspondant (12, 13) et pouvant coopérer avec une broche fixe ou une autre butée (19).

28. Appareil selon la revendication 3, dans lequel les vannes (3, 4) peuvent fonctionner sans ou essentiellement sans chevauchement entre les périodes pendant lesquelles un débit de liquide augmenté est rendu possible.

29. Appareil selon la revendication 25 et selon la revendication 13, dans lequel ledit moyen de commande (52) peut être actionné pour provoquer la rotation des éléments rotatifs (12, 13) jusqu'à la première position dans laquelle la face (16) de chaque élément rotatif (12, 13) est alignée avec le tube et le mouvement des éléments déformant (26, 27) jusqu'à la première position, de façon à permettre que le tube (1) puisse être enlevé desdits passages (8, 9) et de la chambre.

30. Appareil selon la revendication 19 et correspondant à la revendication 3, dans lequel ledit moyen de commande (52) peut être actionné de façon que les deux vannes (3, 4) limitent le débit de liquide dans le tube (1) quand ladite fermeture (41) est ouverte.

31. Appareil selon la revendication 30, dans lequel ledit moyen de commande (52) peut être actionné en ouvrant la fermeture (41) de manière que l'élément mobile (27) se déplace vers ladite première position relative après que les vannes (3, 4) ont limité ledit débit.

32. Appareil selon la revendication 31, dans lequel le moyen de commande (52) peut être actionné pour fermer la fermeture (41) en provoquant le déplacement de l'élément mobile (27) jusqu'à ladite deuxième position relative avec la vanne en aval limitant le débit de liquide et la vanne en amont permettant un débit de liquide augmenté et ensuite pour déplacer les deux vannes (3, 4) restant dans les conditions précitées jusqu'à un position de démarrage prédéterminée après laquelle le moyen de commande (52) peut être actionné pour que le moyen en amont limite le débit de liquide et que la vanne en aval permette un débit de liquide augmenté et pour provoquer le recommencement du cycle de fonctionnement de ladite vanne (3, 4) et du mouvement alternatif desdits éléments (26, 27).

33. Appareil selon la revendication 31, comportant un moyen (46) contrôlable par l'opérateur, accessible à l'opérateur uniquement quand ladite fermeture (41) est ouverte et actionnable après que l'élément mobile (27) s'est déplacé jusqu'à ladite première position relative pour l'ouverture de ladite fermeture (41).

34. Appareil selon la revendication 23 et correspondant à la revendication 18, dans lequel chaque élément rotatif (12, 13) et l'élément mobile (27) peuvent être entraînés par des moteurs électriques correspondants (20, 21, 37) qui sont commandés par un microprocesseur (52).

35. Appareil selon la revendication 34 et dans lequel au moins le moteur (37), prévu pour entraîner l'élément mobile (27), est associé à un encodeur (49) qui produit un signal de sortie indiquant la position ou l'extension du déplacement de l'élément (27) entraîné par ce moteur (37), le moyen (51) étant prévu pour envoyer les signaux ainsi produits audit microprocesseur (52) pour être utilisés comme signaux de référence pour la programmation de la création de signaux de commande du moteur par ledit microprocesseur (52).
